# EUROPEAN PATENT APPLICATION

(11) **EP 2 489 346 A1**
(43) Date of publication of application: **22.08.2012**
(21) Application number: 11152208.2
(22) Date of filing: 26.01.2011
(51) Int. Cl.: A61K 9/00, A61K 9/48, A61K 33/06, A61K 33/42, A61K 38/39, A61K 38/43, A61P 19/10

(54) **Food supplement and injectable material for prophylaxis and therapy of osteoporosis and other bone diseases**

(71) Applicant: NanotecMARIN GmbH, 55128 Mainz (DE)
(72) Inventor: Müller, Dr. Werner E.G., 65203 Wiesbaden (DE); Wang, Dr. Xiaohong, 55218 Ingelheim (DE); Schröder, Dr. Heinz C., 65189 Wiesbaden (DE)
(74) Representative: Krauss, Jan

(57) **Abstract**

The invention concerns the application of inorganic polyphosphates (polyP) and complexes of polyP and calcium [polyP (Ca²⁺ complex)] for prophylaxis and treatment of osteoporosis and other bone diseases by inducing hydroxyapatite formation and decreasing osteoclastogenesis. PolyP and polyP (Ca²⁺ complex) can be used both as a drug or food supplement and as a material to be injected into bone tissue.

## Description

The invention relates to the application of inorganic polyphosphates (polyP) and complexes of polyP and calcium [polyP (Ca²⁺ complex)] for prophylaxis and treatment of osteoporosis and other bone diseases by inducing hydroxyapatite formation and decreasing osteoclastogenesis. PolyP and polyP (Ca²⁺ complex) can be used both as a drug or food supplement and as a material to be injected into bone tissue.

### Background of Invention

Osteoporosis is the most common metabolic disease of bones. The costs caused by osteoporosis to the health systems are tremendous and will further increase as a result of the demographic development in many countries worldwide. Osteoporosis is associated with an enhanced risk of bone fracture. The increased bone fragility is caused by a reduced mineral density of bone tissue and by a deterioration of the bone microarchitecture. Osteoporosis is classified as either primary or secondary. Primary osteoporosis is subdivided into Type I (postmenopausal) and Type II (age-related or senile) osteoporosis. Type I or postmenopausal osteoporosis is most common in women after menopause due to estrogen deficiency. Type II or senile osteoporosis may develop in both females and males. Secondary osteoporosis may occur as a result of hormonal disorders (e.g., hyperparathyroidism) or treatment of patients with glucocorticoids (steroid-induced osteoporosis). In addition, nutritional factors may be involved in the development of osteoporotic disorders.

In osteoporosis the deterioration of the microarchitecture of bone tissue in particular concerns the trabecular bone. As a result, shrinkage of cortical width, rarefaction of the trabecular network and increased occurrence of disconnected trabeculae due to increased osteoclastic resorption may occur. Therefore, hip fractures and vertebral fractures (compression fractures) are often observed in osteoporotic patients.

The mechanism underlying the pathogenesis of osteoporosis is an imbalance between bone resorption and bone formation. The cells responsible for mineralization of bone tissue are osteoblasts, and the cells responsible for bone resorption are osteoclasts, which inhabit the bone surface. The differentiation of pre-osteoclasts to mature osteoclasts and the activation of these cells are regulated by various factors belonging to the tumor necrosis factor (TNF) and TNF receptor superfamily, including RANKL (receptor activator for nuclear factor κB ligand) and osteoprotegerin (OPG). RANKL is produced by osteoblasts; it binds to and thereby stimulates its receptor RANK (receptor activator of nuclear factor κB) on osteoclast precursor cells. OPG binds RANKL, resulting in an inhibition of bone resorption. The RANKL/RANK interaction plays a fundamental role in the differentiation and maintenance of osteoclast activity, and thus in the development of osteoporosis.

In therapy of osteoporosis, several medications are currently used, including bisphosphonates, Selective Estrogen-Receptor Modulators (SERMs; Raloxifene), Teriparatide (recombinant parathyroid hormone), strontium ranelate, RANKL inhibitors (Denosumab, a monoclonal antibody mimicking the activity of OPG), and calcium and vitamin D (used as supplements).

Bisphosphonates are synthetic analogous of pyrophosphate; the oxygen atom of the P-O-P bond of pyrophosphate has been replaced by carbon. The resulting P-C-P group makes the bisphosphonates resistant to enzymatic hydrolysis. Various compounds are available (e.g., alendronate, risedronate, ibandronate, and zoledronate).

### Polyphosphate

Inorganic polyphosphates (polyP) are linear polymers of orthophosphate (Pᵢ) residues linked by energy-rich phosphoanhydride bonds. These polymers may range in size of up to several thousands Pᵢ residues. They are present in bacteria, fungi, and algae, but also in higher plants and animals, including human cells and tissues; they are also found extracellularly in human blood plasma (e.g., Lorenz B, Münkner J, Oliveira MP, Kuusksalu A, Leitão JM, Müller WEG, Schröder HC. Changes in metabolism of inorganic polyphosphate in rat tissues and human cells during development and apoptosis. Biochim Biophys Acta 1335:51-60, 1997; Leyhausen G, Lorenz B, Zhu H, Geurtsen W, Bohnensack R, Müller WEG, Schröder HC. Inorganic polyphosphate in human osteoblast-like cells. J Bone Mineral Res 13:803-812, 1998).

PolyP is stable over a wide temperature and pH range; it is readily soluble in water in millimolar concentrations at chain lengths below 100 phosphate units (Kulaev IS, Vagabov VM, Kulakovskaya TV. The Biochemistry of Inorganic Polyphosphates. Chichester, England: John Wiley & Sons, Ltd pp 1-277, 2004).

The biological function of polyP has mainly been studied in microorganisms (reviewed in: Kulaev IS, Vagabov VM, Kulakovskaya TV. The Biochemistry of Inorganic Polyphosphates. Chichester, England: John Wiley & Sons, Ltd pp 1-277, 2004) and more recently also in animals (reviewed in: Schröder HC, Lorenz B, Kurz L, Müller WEG: Inorganic polyP in eukaryotes: enzymes, metabolism and function. In: Schröder HC, Müller WEG, eds, Inorganic Polyphosphates - Biochemistry, Biology, Biotechnology. Prog. Mol. Subcell. Biol. 23:45-81, 1999; Rao NN, Gómez-Garcia MR, Kornberg A. Inorganic polyphosphate: Essential for growth and survival. Annu Rev Biochem 78:605-647, 2009).

PolyP may act as:
■ storage molecule for energy-rich phosphate,
■ chelator for Ca²⁺ or other divalent cations,
■ counterion for basic amino acids,
■ donor for sugar and adenylate kinase,
■ modulator of gene expression,
■ component of cell membrane complexes with poly-ß-hydroxybutyrate / Ca²⁺,
■ donor of Pᵢ for certain sugar kinases,
■ inducer of apoptosis, and
■ modulator of cellular response to osmotic and pH stress.

In bacteria, polyP is synthesized from ATP by the polyphosphate kinase (Akiyama M, Crooke E, Kornberg A. The polyphosphate kinase gene of Escherichia coli. Isolation and sequence of the ppk gene and membrane location of the protein. J Biol Chem 267:22556-22561, 1992). The degradation of polyP in both prokaryotic and eukaryotic cells is catalyzed by several endo- and exopolyphosphatases (e.g., Lorenz B, Müller WEG, Kulaev IS, Schröder HC. Purification and characterization of an exopolyphosphatase activity from Saccharomyces cerevisiae. J Biol Chem 269:22198-22204, 1994).

The current state of knowledge on polyP has been reviewed in:
- Kornberg A. Inorganic polyphosphate: a molecule of many functions. In: Schröder HC, Müller, WEG (Eds) Inorganic polyphosphates: biochemistry, biology, biotechnology. Progress in Molecular and Subcellular Biology; Vol 23. Springer: Berlin; pp 1-26, 1999.
- Kulaev IS, Vagabov VM, Kulakovskaya TV. The Biochemistry of Inorganic Polyphosphates. Chichester, England: John Wiley & Sons, Ltd pp 1-277, 2004.
- Omelon SJ, Grynpas MD. Relationships between polyphosphate chemistry, biochemistry and apatite biomineralization. Chem Rev 108:4694-4715, 2008.
- Rao NN, Gómez-Garcia MR, Kornberg A. Inorganic polyphosphate: Essential for growth and survival. Annu Rev Biochem 78:605-647, 2009.
- Schröder HC, Müller WEG (Eds). Inorganic Polyphosphates. Biochemistry. Biology. Biotechnology. Vol 23, Springer Verlag: Berlin Heidelberg, 1999.

The following publications are relevant with regard to the application of polyP in human:
- Leyhausen G, Lorenz B, Zhu H, Geurtsen W, Bohnensack R, Müller WEG, Schröder HC. Inorganic polyphosphate in human osteoblast-like cells. J Bone Mineral Res 13:803-812, 1998.
- Lorenz B, Münkner J, Oliveira MP, Kuusksalu A, Leitão JM, Müller WEG, Schröder HC. Changes in metabolism of inorganic polyphosphate in rat tissues and human cells during development and apoptosis. Biochim Biophys Acta 1335:51-60, 1997.
- Schröder HC, Kurz L, Müller WEG, Lorenz B. Polyphosphate in bone. Biochemistry (Moscow) 65:296-303, 2000.

These publications do not describe the suppression of osteoclastogenesis by polyP and polyP (Ca²⁺ complex) and the stimulating effect of the Ca²⁺ complex of polyP on hydroxyapatite (HA) formation, according to this invention, which are essential for their application in therapy and prophylaxis of osteoporosis and related diseases.

The following patent applications are relevant with regard to the analysis of the chain length of polyP and its interaction with metal oxides:
- German Patent No. DE19703025C2. Verfahren zur Messung von Konzentrationen sowie zur kontinuierlichen oder diskontinuierlichen Messung des Umsatzes und der Geschwindigkeit enzymatischer oder nichtenzymatischer Reaktionen von Pyrophosphaten und/oder linearen Polyphosphaten unter Ausschlulß von zyklischen Polyphosphaten als auch von Orthophosphaten in einer Probe sowie Anwendung des Verfahrens. Inventors: Lorenz B, Müller WEG, Schröder HC.
- Patent application: DE 4309248 A1**.** Modifikation von Metalloxiden mit Polyphosphaten oder ähnlichen Verbindungen und ihre Anwendung. Inventors: Lorenz B, Marmé S, Unger K, Schröder HC, Müller WEG.

The following patents or patent applications on formation of biosilica, which may be used in combination with polyP or polyP (Ca²⁺ complex), and its application in biomedicine and dentistry are relevant:

European Patent No. EP1320624, United States Patent No. US7169589B2**,** German Patent No. DE10037270**,** Chinese Patent No. CN01813484.X**,** New Zealand Patent No. NZ523474, Australia Patent No. AU2001289713. Silicatein-mediated synthesis of amorphous silicates and siloxanes and their uses. Inventors: Müller WEG, Lorenz A, Krasko A, Schröder HC; national phases: NO20030407; JP2002516336; CA2414602**.**

United States Patent No. US6670438B1**.** Methods, compositions, and biomimetic catalysts for in vitro synthesis of silica, polysilsequioxane, polysiloxane, and polymetallo-oxanes. Inventors: Morse DE, Stucky GD, Deming, TD, Cha J, Shimizu K, Zhou Y.

German Patent No. DE10246186**.** In vitro and in vivo degradation or synthesis of silicon dioxide and silicones, useful e.g. for treating silicosis or to prepare prosthetic materials, using a new silicase enzyme. Inventors: Müller WEG, Krasko A, Schröder HC.

European Patent No. EP1546319**.** Abbau und Modifizierung von Silicaten und Siliconen durch Silicase und Verwendung des reversiblen Enzyms. Inventors: Müller WEG, Krasko A, Schröder HC.

Patent application: EP1740707; US11579019; DE10352433.9; CA2565118**;** JP2007509991**.** Enzym- und Template-gesteuerte Synthese von Silica aus nicht-organischen Siliciumverbindungen sowie Aminosilanen und Silazanen und Verwendung. Inventors: Schwertner H, Müller WEG, Schröder HC.

Patent application: EP09005849.6. Use of silintaphin for the structure-directed fabrication of (nano)composite materials in medicine and (nano)technology. Inventors: Wiens M, Müller WEG, Schröder HC, Wang X.

Patent applications: DE102004021229.5**;** EP2005004738; US11579020**;** JP2007509992; CA2565121. Enzymatic method for producing bioactive, osteoblast-stimulating surfaces and use thereof. Inventors: Müller WEG, Schwertner H, Schröder HC.

Patent applications: US 60839601; EP 2007007363**.** Biosilica-adhesive protein nano-composite materials: synthesis and application in dentistry. Inventors: Müller WEG, Schröder HC,Geurtsen WK.

Patent application: PCT/US2009/005302**.** Compositions, oral care products and methods of making and using the same. Inventors: Miller J, Höfer H, Geurtsen W, Lücker P, Wiens M, Schröder HC, Müller WEG.

### Summary of the Invention

This invention concerns the unexpected property of inorganic polyphosphate (polyP) and polyP supplied in a stoichiometric ratio of 2 moles of polyP : 1 mole of CaC1₂ [polyP (Ca²⁺ complex)] to impair osteoclastogenesis. At concentrations > 10 µM polyP (Ca²⁺ complex) slows down the progression of bone resorbing cells (e.g., osteoclast-like RAW 264.7 cells) to functional osteoclasts, as measured by the expression of tartrate-resistant acid phosphatase (TRAP), a marker protein for terminally differentiated osteoclasts. In addition, according to this invention, polyP (Ca²⁺ complex), but not polyP, induces HA formation in bone forming osteoblasts. This unexpected effect can be observed already at concentrations as low as 10 µM, as demonstrated by staining with Alizarin Red S and scanning electron microscopy. Both polyP and polyP (Ca²⁺ complex) were found to be non-toxic to all cells tested (including osteoblast-like SaOS-2 cells and RAW 264.7 cells) at concentrations of up to 100 µM and more. Furthermore, polyP (Ca²⁺ complex) triggers in osteoblasts the expression of bone morphogenetic protein 2 (BMP2), an inducer of bone formation. This cytokine is involved in maturation of HA-forming cells. Finally it is described that in osteoclasts polyP (Ca²⁺ complex) inhibits phosphorylation of IκBα by the IκBα kinase that mediates activation of NF-κB during RANKL-caused (pre)osteoclast differentiation. This invention also involves various formulations of polyP- and polyP (Ca²⁺ complex)-containing materials, which can be administered either as a drug or food supplement or by percutaneous injection.

Thus, according to this invention, polyP (Ca²⁺ complex) displays a dual effect on bone metabolizing cells: (a) it promotes HA formation in bone forming cells (osteoblasts) and (b) it impairs maturation of osteoclast precursor cells. This invention represents a significant progress beyond the state-of-the-art, because it describes a new strategy for prophylaxis and/or treatment of osteoporosis and other bone diseases.

A further aspect of this invention concerns the combination of polyP or polyP (Ca²⁺ complex) with silicic acid or polysilicic acid, as well as their salts (in particular calcium salts), stabilized complexes, or enzymatically synthesized forms (biosilica). In earlier studies, the inventors had been demonstrated that in osteoblasts biosilica not only induces HA formation (EP 2005004738; US11579020) but also stimulates osteoprotegerin (OPG) synthesis (EP10167744.1; via up-regulation of BMP2 expression), thus counteracting the function of receptor activator of the NF-kB ligand (RANKL), i.e. the maturation of pre-osteoclasts and the activation of osteoclasts. Because the OPG : RANKL ratio is crucial in the development of osteoporosis, this combination brings added value to the application polyP (Ca²⁺ complex) alone.

In the experiments described below, osteoblast-like bone forming SaOS-2 cells and osteoclast-like bone resorbing RAW 264.7 cells have been used. These cell lines express the key proteins RANK, RANKL, OPG, as well as BMP2 and TRAP.

SaOS-2 (sarcoma osteogenic) is a nontransformed cell line that is derived from primary osteosarcoma cells having a limited differentiation capacity.

RAW 264.7 cells are monocyte-macrophage precursors of osteoclast-like cells. The progression of these cells to functional osteoclasts can be monitored by measuring the expression of TRAP (Filgueira L. Fluorescence-based staining for tartrate-resistant acidic phosphatase (TRAP) in osteoclasts combined with other fluorescent dyes and protocols. J Histochem Cytochem 52:411-414, 2004).

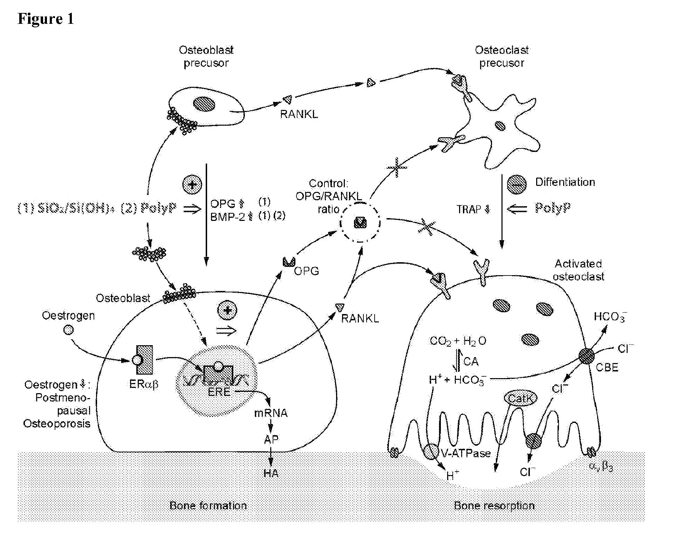

A schematic presentation of the effect of polyP (Ca²⁺ complex), according to this invention, on (a) osteoclastogenesis and (b) bone HA formation in osteoblasts is given in Figure 1. In addition, the RANK/RANKL/OPG system and the effect of the biosilica, which may be used in combination with polyP (Ca²⁺ complex), are shown.

### Material consisting of polyP or polyP (Ca²⁺ complex) for prophylaxis or treatment of osteoporosis and other bone disorders

This invention concerns a material to be used as a drug or food supplement or an injectable material, consisting of inorganic polyphosphate (polyP) or complexes of polyP and calcium [polyP (Ca²⁺ complex)] or combinations thereof. The chain length of the polyP molecules or of the polyP molecules of the polyP (Ca²⁺ complex) can be in the range between 2 to 1000 phosphate units, but also chain length between 10 to 100 phosphate units, or 100 to 1000 phosphate units, or more narrow ranges of chain lengths are possible for specific application protocols.

A further aspect of this invention concerns a material to be used as a drug or food supplement or an injectable material, consisting of a combination of polyP or polyP (Ca²⁺ complex) and monomeric silicic acid (orthosilicic acid) or polymeric silicic acid (silica). The monomeric or polymeric silicic acid may be present as a calcium salt.

The monomeric or polymeric silicic acid used in combination with polyP or polyP (Ca²⁺ complex) may also be present in a stabilized form (for example: hydronium stabilized, choline stabilized, or protein-stabilized).

Moreover, polymeric silicic acid can be added that had been formed by an enzyme or protein involved in the metabolism of biosilica (amorphous, hydrated silicon oxide). Such enzymes or proteins may be silicatein, silicase, silintaphin-1, silicatein-silintaphin-1 fusion proteins or combinations thereof.

In addition, these combinations may include a suitable substrate such as orthosilicic acid or tetraethyoxysilane.

The enzyme or protein additive used in combination with polyP or polyP (Ca²⁺ complex) may be provided with an N-terminal or C-terminal apatite (calcium phosphate)-binding or silica-binding oligocationic or oligoanionic tag, such as oligoglutamic acid, oligoaspartic acid or oligolysine (allowing coordination/complex interactions between carboxyl groups and Ca²⁺ ions of hydroxapatite), or a multiple thiol groups carrying tag, such as oligocysteine (allowing coordination/complex interactions with iron ions). This enzyme or protein additive may be produced synthetically or using a prokaryotic or eukaryotic expression system.

The polyP or polyP (Ca²⁺ complex) may be encapsulated in an organic polymer. This polymer may consist of shellac, alginate, poly(lactic acid), or poly(D,L-lactide)/polyvinyl pyrrolidone-based microspheres.

The use of shellac has the advantage that because of its alkaline properties, it prevents dissolution of the encapsulated material [polyP or polyP (Ca²⁺ complex) without or with further additives] in the stomach and allows for a timed enteric or colonic release.

In addition, it is possible also to encapsulate combinations of polyP or polyP (Ca²⁺ complex) and monomeric or polymeric silicic acid or the silica-metabolic enzymes, proteins, and substrates described in an organic polymer, consisting of shellac, alginate, or poly(lactic acid), or poly(D,L-lactide)/polyvinyl pyrrolidone-based microspheres.

A further aspect of this invention concerns a material, consisting of polyP or polyP (Ca²⁺ complex) or their combinations, which had been bound to silica or HA nanoparticles.

Tagged silicatein, silicase or silintaphin-1 molecules can be bound via their protein tag to silica or HA nanoparticles using state-of-the-art methods (e.g. EP10167744.1).

Based on its properties, the material according to this invention can be used as a food supplement for prophylaxis or therapy of osteoporosis or other bone disorders. This material can be used as a supplement to yoghurt or other milk products.

Furthermore, the material according to this invention allows the formulation of an orally or parenterally-administrable drug for therapy or prophylaxis of osteoporosis or other bone disorders.

This material can also be used for percutaneous injection into fractured bone tissue or for prophylactic stabilization of vertebral bodies in osteoporotic patients or patients with other bone disorders.

Bisphosphonates may inhibit the degradation of polyP (Leyhausen G, Lorenz B, Zhu H, Geurtsen W, Bohnensack R, Müller WE, Schröder HC. Inorganic polyphosphate in human osteoblast-like cells. J Bone Miner Res 13:803-812, 1998). Therefore, administration of bisphosphonates in addition to the inventive material and its various formulations may be of advantage in prophylaxis or treatment of osteoporosis and related bone diseases.

Figure 1 shows a schematic presentation of the effect of polyP (Ca²⁺ complex), according to this invention, on (a) osteoclastogenesis and (b) bone HA formation in osteoblasts. The dual activity of polyP (Ca²⁺ complex) [(a) impairment of osteoclastogenesis (effect on osteoclasts) and (b) increase in bone HA formation (effect on osteoblasts)] provides the basis for the biomedical potential of polyP (Ca²⁺ complex) in therapy and prophylaxis of osteoporosis. Figure 1 also depicts the effect of biosilica on osteoblasts. Biosilica stimulates the expression / release of OPG but does not affect the expression of RANKL, thus sequestering RANKL which becomes unavailable to bind to RANK, its receptor on the (pre)ostecloasts. Based on these different mechanisms of polyP (Ca²⁺ complex) and biosilica (or silicic acid), an increased anti-osteoporotic activity of the inventive material will result.

The studies underlying this invention have been performed with two cell lines; either with SaOS-2 cells as a model system for osteoblasts, having the capacity of HA formation or with RAW 264.7 cells which show characteristics of HA-resorbing osteoclasts. The results are described in the following.

### Effect of polyP on cell viability of SaOS-2 and RAW 264.7 cells

Both, SaOS-2 cells growing in McCoy's medium/5% FCS and RAW 264.7 cells cultured in DMEM/10% FCS, were incubated with increasing concentrations of polyP (Ca²⁺ complex).

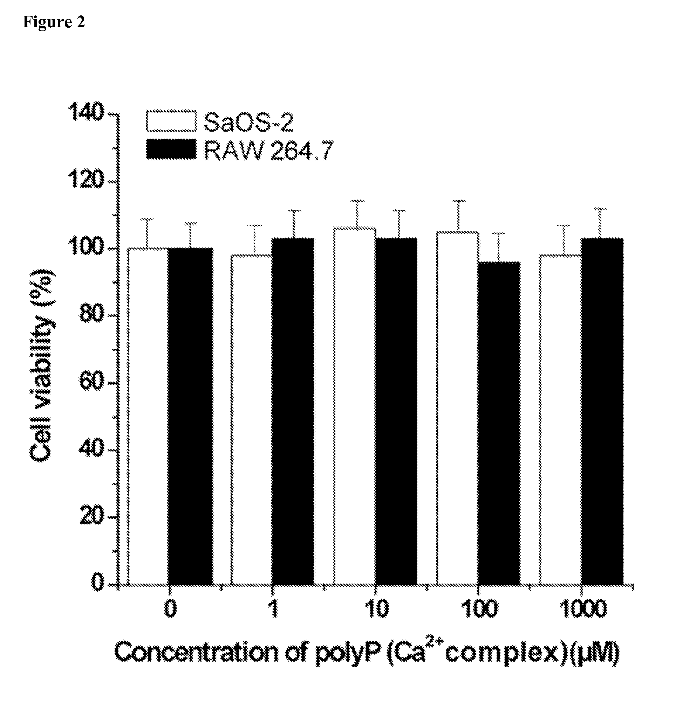

As summarized in Figure 2 this polymer did not cause any significant reduction of cell viability within the concentration range of 1 to 1000 µM during an incubation period of 5 days.

### PolyP-induced mineralization in SaOS-2 cells: alizarin red staining

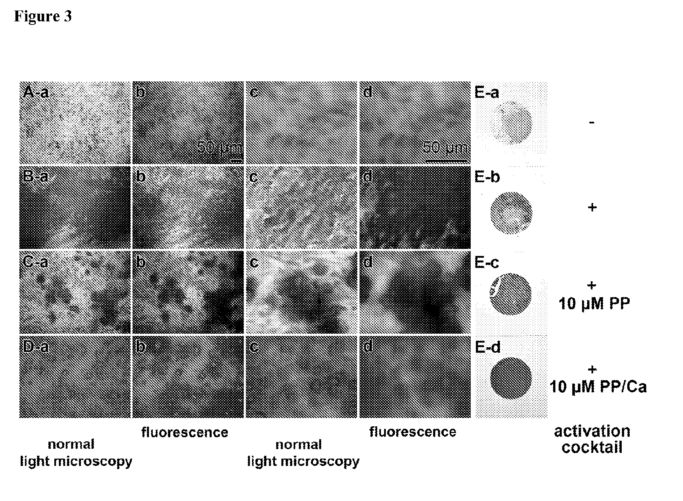

To study the effect of polyP (Ca²⁺ complex) on the formation of HA, SaOS-2 cells were incubated for 10 days on plastic cover slips in 24-well plates using McCoy's medium/10% FCS in the absence or presence of AC (see under Methodology). Additional cultures grown in AC were exposed to 10 µM polyP or 10 µM polyP (Ca²⁺ complex) for 10 days. HA formation was determined by using the Alizarin Red S (AR-S) staining technique. The intensity of the staining was lowest in cultures grown in the absence of AC (Figure 3E-a). In contrast, cultures growing in the presence of AC showed a significantly intensified staining reflecting a higher level of HA mineralization (Figure 3E-b). If the cultures were treated with AC and 10 µM polyP no striking difference in the intensity of AR-S staining, compared with cultures grown with AC only, was observed (Figure 3E-c). However, if the cultures were grown in medium containing AC and 10 µM polyP (Ca²⁺ complex) a strong and almost homogenous staining of the cell layer could be visualized (Figure 3E-d). Thus an extensive mineralization can only be recorded if polyP is added to the cells in a 2:1 stoichiometric ratio with the Ca²⁺ ions [polyP (Ca²⁺ complex)].

Digital light microscopy revealed that the samples grown in McCoy's medium/FCS showed only a scattered staining of the cell layer (Figure 3A-a). Likewise low was the red staining if the samples were examined by red/green emitting fluorescence light (Figure 3A-b); those images were computed by an overlay of the images obtained by red or green fluorescence. The intensities of the red patches were low and the areas lighting up were small. The same distribution was seen if the samples were inspected at a higher magnification (Figure 3A-c and d). A distinct increase in the red intensities was seen if the cells were grown in the presence of AC and then stained with AR-S to monitor HA deposition (Figure 3B). Under the conditions used, the red patches (Figure 3B-a and c) as well as the red fluorescence areas (Figure 3B-b and d) strongly increased and occupied over 50% of the visual fields. If the cells were incubated for 7 days in the presence of both, AC and 10 µM polyP the red patches/red fluorescence areas were smaller (Figure 3C-a and b; Figure 3C-c and d) if compared with the fields stained with AR-S in cultures grown with AC only (Figure 3B). However, if the cultures were incubated with AC together with 10 µM polyP (Ca²⁺ complex) an almost complete red staining, reflecting an intensive HA mineralization, was seen. This result was found both by application of normal/incandescent (Figure 3D-a and c) and with fluorescent light (Figure 3D-b and d).

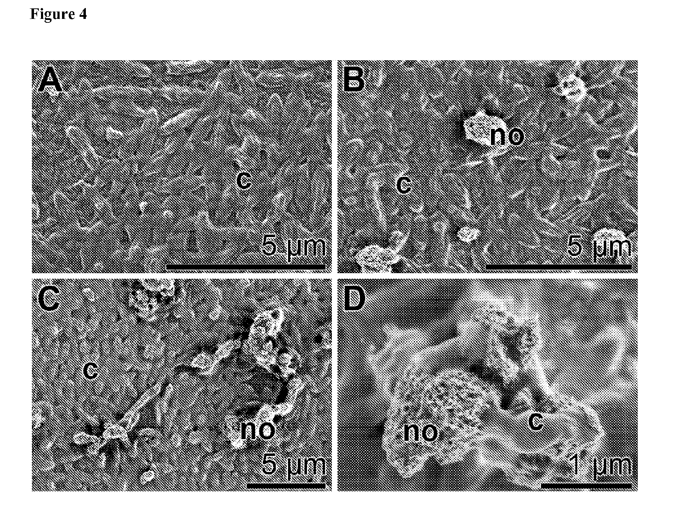

The differential extent of mineralization of SaOS-2 cells grown in medium/AC supplemented with 10 µM polyP or 10 µM polyP (Ca²⁺ complex) could also be documented by SEM analyses. Cultures grown for 5 days in medium in the absence of AC (Figure 4A) showed (almost) no HA deposits on the cells. However if they were grown in the presence of AC and 10 µM polyP HA nodules were frequently seen (Figure 4B). However, if they were cultivated in medium/AC and 10 µM of polyP (Ca²⁺ complex) a significant increase in the clustering of HA deposits/nodules was seen (Figure 4C). At higher magnification it was visible that the growing nodules were intimately surrounded by cell protrusions (Figure 4D).

### PolyP-induced mineralization in SaOS-2 cells: quantitative measurement

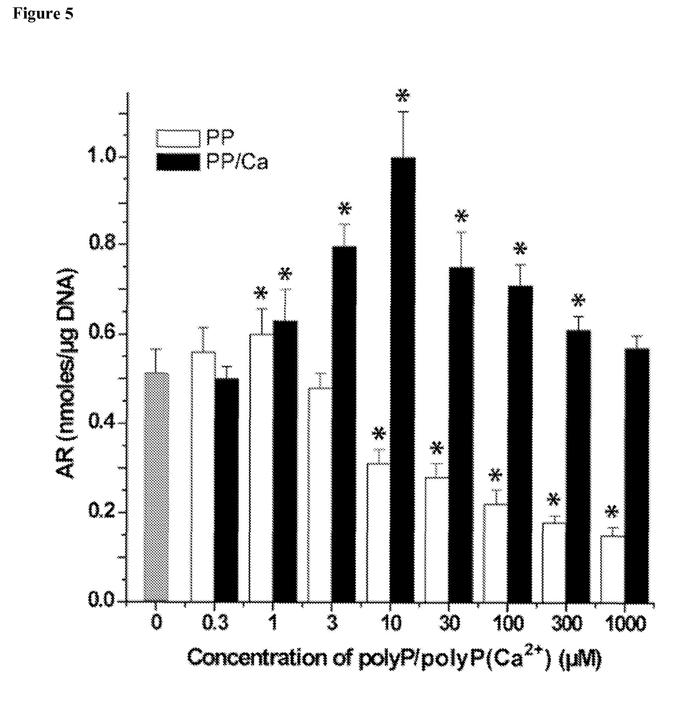

A quantitative assessment of HA formation, based on AR-S reaction was achieved by a spectroscopic technique; the optical density was correlated with the cell number, as measured by DNA concentration in the assays. The SaOS-2 cells were grown in the presence of AC for 7 days. In the absence of any polyP / polyP (Ca²⁺ complex) the extent of AR-S-caused increase in optical density (at 405 nm) was 0.52 nmoles/u.g DNA (Figure 5). If either polyP or polyP (Ca²⁺ complex) was added at a concentration of 1 µM a significant increase in the optical density was seen. The values were correlated to the cell number/DNA content in the assays. Highest intensity of AR-S staining was observed at a concentration of 10 µM polyP (Ca²⁺ complex). However, if polyP instead of polyP (Ca²⁺ complex) was added a decrease in mineralization was measured. The differences became significant at concentrations of > 10 µM polyP; the highest reduction was recorded at 100 µM polyP.

### Effect of polyP on the expression of BMP2 in SaOS-2 cells

BMP2 is a member of the transforming growth factor-*β* (TGF*β*) superfamily. It has been shown that differentiation of osteoblasts requires the expression of BMP2 (Tanaka H, Nagai E, Murata H, Tsubone T, Shirakura Y, Sugiyama T, Taguchi T, Kawai S. Involvement of bone morphogenic protein-2 (BMP-2) in the pathological ossification process of the spinal ligament. Rheumatology 40:1163-1168, 2001).

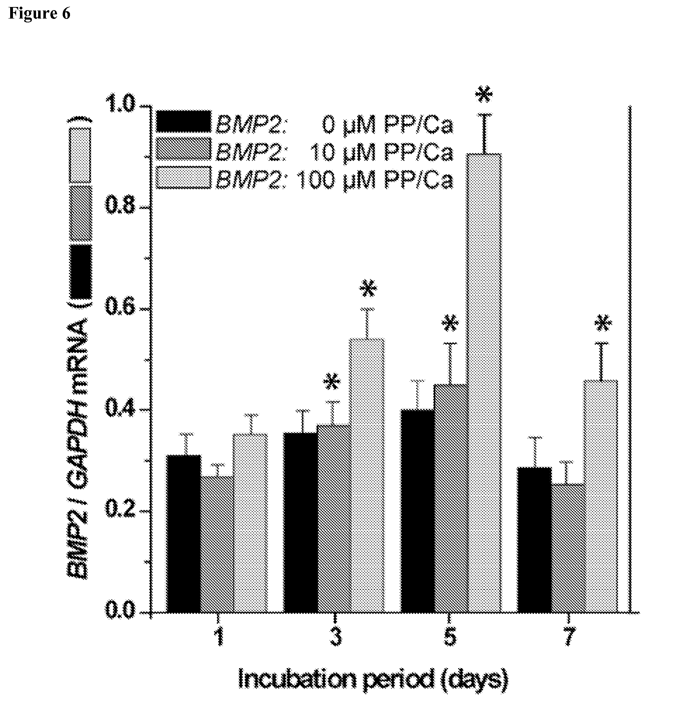

The expression level of *BMP2* in response to polyP (Ca²⁺ complex) was determined by qRT-PCR analysis. SaOS-2 cells were incubated in mineralization medium (McCoy's medium/AC) for 1-7 days. Increasing concentrations of polyP (Ca²⁺ complex), 0 to 100 µM, were added to the cultures at the beginning of the experiments. After termination RNA was extracted from the cultures and subjected to qRT-PCR. The expression of the housekeeping gene *GAPDH* was used as reference. As shown in Figure 6 the expression levels of *BMP2* significantly increased 3 to 5 days after addition of polyP (Ca²⁺ complex). This increase was especially pronounced at a polyP (Ca²⁺ complex) concentration of 100 µM. After 7 days, the expression level decreased but remained still significantly elevated in the assays containing 100 µM polyP (Ca²⁺ complex). In contrast, the expression of BMP2 did not significantly change in cells that were grown in the absence of polyP (Ca²⁺ complex); Figure 6.

### Inhibition of osteoclastogenesis by polyP

RAW 264.7 cells were used to evaluate the effect of polyP (Ca²⁺ complex) on osteoclastogenesis. This osteoclast-like monocytic cell line has the potency to readily differentiate into osteoclasts when exposed to recombinant RANKL (Tsuda E, Goto M, Mochizuki S, Yano K, Kobayashi F, Morinaga T, Higashio K. Isolation of a novel cytokine from human fibroblasts that specifically inhibits osteoclastogenesis. Biochem Biophys Res Commun 234:137-142, 1997). This cell line has successfully been used as a model for studies of osteoclastogenesis *in vitro* (Wittrant Y, Theoleyre S, Couillaud S, Dunstan C, Heymann D, Rédini F. Relevance of an in vitro osteoclastogenesis system to study receptor activator of NF-kB ligand and osteoprotegerin biological activities. Expt Cell Res 293:292-301, 2004).

The expression of TRAP was determined as a suitable marker for analysis of the differentiation of osteoclast precursor cells to mature osteoclasts.

TRAP is a modulator of bone resorption and its increased expression is associated with osteoporosis and other bone diseases (Oddie GW, Schenk G, Angel NZ, Walsh N, Guddat LW, de Jersey J, Cassady AI, Hamilton SE, Hume DA. Structure, function, and regulation of tartrate-resistant acid phosphatase. Bone 27:575-584, 2000).

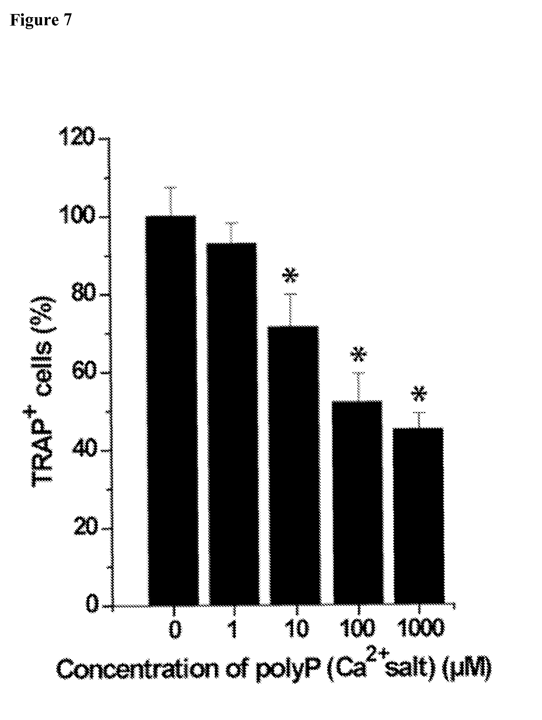

It is well established that inhibition of differentiation of osteoclast can be monitored by using RAW 264.7 cells that are induced to differentiate by soluble RANKL. The inventors found that the number of TRAP⁺ cells in cultures, induced by RANKL, was significantly reduced in the presence of polyP (Ca²⁺ complex). Already at the low concentration of 10 µM polyP (Ca²⁺ complex) a significant reduction of TRAP⁺ cells is determined (32% inhibition of the number of TRAP⁺ cells; incubation period, 6 days); at higher concentrations the decrease of TRAP⁺ cells even increases and reaches values of over 55% (Figure 7).

### PolyP impairs RANKL-induced IκBα phosphorylation in RAW 264.7 cells

In osteoclasts, the IκBα kinase is activated by treatment with RANKL; this process is required for NF-κB activation and induction of osteoclastogenesis (Lee ZH, Kim HH. Signal transduction by receptor activatior of nuclear factor kappa B in osteoclasts. Biochem Biophys Res Commun 305:211-214, 2003; Sung B, Murakami A, Oyajobi BO, Aggarwal BB. Zerumbone abolishes RANKL-induced NF-κB activation, inhibits osteoclastogenesis, and suppresses human breast cancer-induced bone loss in athymic nude mice. Cancer Res 69:1477-1484, 2009).

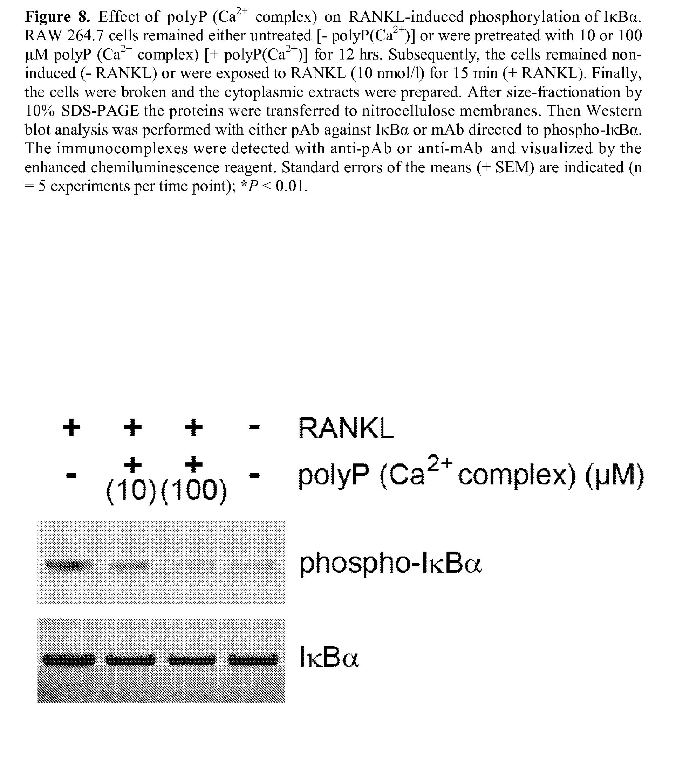

In order to assess the effect of polyP on phosphorylation of IκBα in RAW 264.7 cells, cultures remained either untreated or were pretreated with 10 µM or 100 µM polyP (Ca²⁺ complex) for 12 hrs. Then the cells were incubated with ALLN in order to inhibit ubiquitination and degradation of IκBα (see Methodology section). Finally, the cultures were exposed to RANKL (Figure 8). Cytoplasmic fractions were prepared and subjected to Western blot analysis. The results showed that in the presence of RANKL the signal of phospho-IκBα was high while in the absence of that ligand only a low level of the phospho-protein was seen (Figure 8). If the cells had been pretreated with polyP (Ca²⁺ complex) for 12 hrs and then exposed to RANKL a significant abrogation of the phosphorylation of IκBα is seen. PolyP (Ca²⁺ complex) alone did not result in a phosphorylation of IκBα. A parallel immunoblot shows that the level of IκBα remained (almost) unchanged (Figure 8).

These results show that RANKL-mediated NF-κB activation is impaired on the level of IκBα kinase.

### Encapsulation of polyP or polyP (Ca²⁺ complex)

The polyP or polyP (Ca²⁺ complex) can be encapsulated in an organic polymer such as shellac, alginate, poly(lactic acid), poly(D,L-lactide)/polyvinyl pyrrolidone-based microspheres or other nontoxic polymers following state-of-the-art procedures. Using the same procedures, it is also possible to encapsulate various combinations of polyP or polyP (Ca²⁺ complex) and monomeric or polymeric silicic acid or the silica-metabolic enzymes, proteins, and substrates.

### Binding of polyP or polyP (Ca²⁺ complex) to silica or HA particles

Polyphosphate-modified silica particles or polyphosphate-modified HA can be prepared using a procedure analogous to that described in: Lorenz B, Marmé S Müller WEG, Unger K, Schröder HC. Preparation and use of polyphosphate-modified zirconia for purification of nucleic acids and proteins. Anal Biochem 216:118-126, 1994, as well as Patent application DE 4309248 A1. Modifikation von Metalloxiden mit Polyphosphaten oder ähnlichen Verbindungen und ihre Anwendung. Inventors: Lorenz B, Marmé S, Unger K, Schröder HC, Müller WEG). In addition, it is possible to immobilize various combinations of polyP or polyP (Ca²⁺ complex) with recombinant proteins, e.g. recombinant Glu-tagged silicatein, to achieve additive / synergistic effects. The obtained "core" particles can subsequently be encased in a biosilica "shell" by incubation with a suitable substrate, e.g. orthosilicate or tetroethoxysilane.

### Application for prophylaxis and/or treatment of osteoporosis and other bone diseases

PolyP or polyP (Ca²⁺ complex) and its various formulations according to this invention as well as its combination with monomeric silicic or polymeric silicic acid (including biosilica) can be applied for treatment of patients with osteoporosis such as primary osteoporosis caused by estrogen deficiency (postmenopausal osteoporosis) and osteoporosis of the elderly (senile osteoporosis) or for prophylaxis of the disease.

The dual activity of polyP or polyP (Ca²⁺ complex) and its various formulations on HA-producing cells (osteoblasts) and HA-resorbing cells (osteoclats), i.e. (a) promotion of osteoblast formation through upregulation of *BMP2* expression followed by increased HA deposition and (b) inhibition of maturation of osteoclast precursor cells to functional osteoclasts by impairment of the NF-κB signaling pathway, may also allow the application of these materials in prophylaxis and/or therapy of other bone diseases, in particular those which are characterized by an increased bone resorption, such as rheumatoid arthritis, Paget's disease, carcinomatosis of bone, periodontitis, hypercalcaemic syndrome, disorders with locally increased resorption, or haematologic disorders (multiple myeloma).

### Application in vertebroplasty / kyphoplasty

PolyP or polyP (Ca²⁺ complex) according to this invention as well as its combination with monomeric silicic or polymeric silicic acid (including biosilica) may be injected (percutaneous injection) into fractured vertebral bodies (e.g., compression fractures in osteoporotic patients) either alone or together with some other material used in vertebroplasty or kyphoplasty such as polymethylmethacrylate (PMMA) by means of one or two bone biopsy needles. The restructured vertebral body is stabilized after hardening of the polymeric material, and finally by replacement of the injected material by the polyP-induced formation of new bone tissue.

### Application as orally or parenterally-administrable drug for prophylaxis or therapy of osteoporosis

PolyP or polyP (Ca²⁺ complex) according to this invention as well as its combination with monomeric silicic or polymeric silicic acid (including biosilica) can also be used for the formulation of an orally or parenterally-administrable drug for prophylaxis or therapy of osteoporosis and other bone disorders. The material can be encapsulated in an organic polymer which may consist of shellac, alginate, or poly(lactic acid), or poly(D,L-lactide)/polyvinyl pyrrolidone-based microspheres or administered in a different form, according to state-of-the-art techniques.

### Application as a food supplement for prophylaxis or therapy of osteoporosis

PolyP or polyP (Ca²⁺ complex) according to this invention as well as its combination with monomeric silicic or polymeric silicic acid (including biosilica) can be administered as a food additive (supplement) for prophylaxis or therapy of osteoporosis and other bone disorders.

### Application as a supplement to yoghurt or other milk products for prophylaxis or therapy of osteoporosis

PolyP or polyP (Ca²⁺ complex) according to this invention as well as its combination with monomeric silicic or polymeric silicic acid (including biosilica) can also be used as a supplement to yoghurt or other milk products for prophylaxis or therapy of osteoporosis or other bone disorders.

### Methodology

### Cells and incubation conditions

SaOS-2 cells (human osteogenic sarcoma cells) can be cultured in McCoy's medium (containing 1 mM CaC1₂; obtained from Biochrom), with 5% heat-inactivated FCS [fetal calf serum], 2 mM L-glutamine, and gentamicin (50 ug/ml) in 25 cm² flasks or in 6-well plates (surface area 9.46 cm²; Orange Scientifique) in a humidified incubator at 37°C and 5% CO₂ using state-of-the-art techniques. Routinely, 3 x 10⁵ cells are added per well (total volume 3 ml).

The murine monocyte/macrophage cell line RAW 264.7 can be purchased from the American Type Culture Collection (Manasas). The cells can be grown in DMEM (Dulbecco's Modified Eagle's Medium; Biochrom) supplemented with 10% heat-inactivated FCS [fetal calf serum], penicillin (100 U/ml), and streptomycin (100 µg/ml) in a humidified atmosphere containing 5% CO₂ at 37°C.

PolyP is added either in the form supplied by the manufacturer (termed here "polyP"), or together with CaCl₂ in a stoichiometric ratio of 2 [polyP] : 1 [CaCl₂] (designated "polyP (Ca²⁺ complex)"). In the experiments described, sodium phosphate glass type 45 (Sigma-Aldrich; average chain length 45) or the Ca²⁺ complex prepared from it had been used.

### Cell proliferation/viability assays

SaOS-2 cells as well as RAW 264.7 cells are seeded at a density of 5x10³ cells per well of a 96-multi-well plate (Orange Scientifique) and cultured for 5 days in McCoy's medium, supplemented with 5% FCS. RAW 264.7 cells are grown in DMEM medium plus 10% heat-inactivated FCS for 5 days. Different concentrations of polyP (Ca²⁺ complex) are added to the cells. After incubation, cell proliferation can be determined by the colorimetric method based on the tetrazolium salt XTT (Cell Proliferation Kit II; Roche).

### Mineralization by SaOS-2 cells in vitro

For the mineralization studies, SaOS-2 cells are incubated on plastic cover slips (Nunc) that had been placed into culture flasks or 24-well plates (14.5 mm diameter; Orange Scientifique) in McCoy's medium, supplemented with 10% FCS, antibiotics, and 50 µM ascorbic acid for 2 days. After attachment, cells are transferred into McCoy's medium/10% FCS which, if not mentioned otherwise, had been supplemented with AC required for initiation of HA formation. The AC (activation cocktail) includes the standard inducers 5 mM β-glycerophosphate, 50 mM ascorbic acid and 10 nM dexamethasone. Simultaneously with AC the polymer is added as polyP or as polyP (Ca²⁺ complex). After the incubation period, the slides are removed and stained with 10% Alizarin Red S (AR-S; Sigma-Aldrich; staining for ossification).

The intensity of AR-S staining can be quantitatively assessed by application of a spectrophotometric assay. Briefly, the cells are transferred into acetic acid. Subsequently, they are removed from the culture dishes by mechanical scraping followed by centrifugation. The supernatant obtained is supplemented with ammonium hydroxide to neutralize the acid and the optical density is read at 405 nm. The amount of bound AR-S is given in moles which had been determined after setting up a calibration curve. Values are normalized to total DNA using the PicoGreen method; calf thymus DNA can be used as a standard.

### Osteoclastic differentiation of RAW 264.7 cells

For the RANKL-induced osteoclastic differentiation the protocol of Choi et al. (Choi HJ, Park YR, Nepal M, Choi BY, Cho NP, Choi SH, Heo SR, Kim HS, Yang MS, Soh Y. Inhibition of osteoclastogenic differentiation by Ikarisoside A in RAW 264.7 cells via JNK and NF-κB signaling pathways. Eur J Pharmacol 636:28-35, 2010) can be used. The RAW264.7 cells are suspended in α-MEM [α-modified minimum essential medium; Biochrom] containing 10% FCS, 2 mM L-glutamate, 100 U/ml penicillin, and 100 ug/ml streptomycin. Then the cells are seeded at a density of 3×10³ cells/well in 96-well culture plates and subsequently cultured with 50 ng/ml recombinant soluble RANKL (e.g., from PeproTech) for 6 days in the presence of different concentrations of polyP (Ca²⁺ complex). The cells are stained for TRAP; e.g., the TRAP staining kit from Wako can be used. Subsequently the cells are cytochemically analyzed.

### Protein expression and Western blot analysis

Inhibition of RANKL-induced phosphorylation of IκBα is performed as follows. RAW 264.7 cells at a density of 5x10⁶ are incubated with 20 µM polyP (Ca²⁺ complex) for 12 hrs, followed by an incubation with ALLN [*N*-acetyl-leu-leu-norleucinal; Roche Diagnostics] (50 µg/ml) for 30 min. Finally the cells are treated with RANKL (10 nmol/1) for 15 min as described by Sung et al. (Sung B, Murakami A, Oyajobi BO, Aggarwal BB. Zerumbone abolishes RANKL-induced NF-κB activation, inhibits osteoclastogenesis, and suppresses human breast cancer-induced bone loss in athymic nude mice. Cancer Res 69:1477-1484, 2009). Prior to sodium dodecyl sulphate polyacrylamide gel electrophoresis (SDS-PAGE) the cytoplasmic fraction is prepared as described by Sung et al. (Sung B, Pandey MK, Aggarwal BB. Fisetin, an inhibitor of cyclin-dependent kinase 6, down-regulates nuclear factor-κB-regulated cell proliferation, antiapoptotic and metastatic gene products through the suppression of TAK-1 and receptor-interacting protein regulated IκBα kinase activation. Mol Pharmacol 71:1703-1714, 2007), which is then fractionated by 10% PAGE. After size fractionation the proteins are transferred to nitrocellulose membranes and incubated either with polyclonal antibodies [pAb] against IκBα (rabbit polyclonal antibodies to IκBα from Santa Cruz Biotechnology) or monoclonal antibodies [mAb] directed to phospho-IκBα (mouse monoclonal antibodies against phospho-IκBα, phosphorylated at Ser32/36, from Cell Signaling Technology). The immunocomplexes are detected with the matching anti-pAb or anti-mAb antibodies (Sigma-Aldrich) and then visualized by the enhanced chemiluminescence reagent (Roche).

### Quantitative real-time RT-PCR analysis

Quantitative real-time PCR (qPCR) analyses to determine the expression level *of BMP2* can be performed using state-of-the-art methods. In short, SaOS-2 cells are incubated in McCoy's medium/15% FCS supplemented with AC for 1-7 days together with polyP (Ca²⁺ complex). Then the cells are harvested and total RNA is extracted and freed from DNA contamination by using DNAse. Subsequently, first-strand cDNA is synthesized by using the M-MLV reverse transcriptase (Promega). Each reaction contains approximately 5 µg of total RNA in the reaction mixture of 40 µl. qPCR experiments can be performed in an iCycler (Bio-Rad), using 1/10 serial dilutions in triplicate. Then, each of the reaction mixtures is diluted as required and 2 µl of the appropriate dilution are employed as template for 30-µl qPCR assays. The reaction is supplemented with SYBR Green master mixture (ABgene) and 5 pmol of each primer [for amplification of *BMP2* and for the housekeeping gene *GAPDH* (glyceraldehyde 3-phosphate dehydrogenase)]. The reactions are run by using the temperature cycles described by Wiens et al. (Wiens M, Wang XH, Schloßmacher U, Lieberwirth I, Glasser G, Ushijima H, Schröder HC, Müller WEG. Osteogenic potential of bio-silica on human osteoblast-like (SaOS-2) cells. Calcif Tissue Intern, 2010. DOI: 10.1007/s00223-O10-9408-6). Fluorescence data are collected at the 80°C step. The following primers can be used for amplification: for the gene *GAPDH* the forward primer 5'-ACTTTGTGAAGCTCATTTCCTGGTA-3' (nt₁₀₁₉ to nt₁₀₄₃) and reverse primer 5'-TTGCTGGGGCTGGTGGTCCA-3' (nt₁₁₁₇ to nt₁₁₃₆, product size 118 bp) and for *BMP2* (NM_001200.2), the primer pair: forward 5'-ACCCTTTGTACGTGGACTTC-3' (nt₁₆₈₁ to nt₁₇₀₀) and reverse 5'-GTGGAGTTCAGATGATCAGC-3' (nt₁₇₈₅ to nt₁₈₀₄, 124 bp). The threshold position is set to 50.0 relative fluorescence units above PCR subtracted baseline for all runs. Expression levels of *BMP2* can be correlated to the reference gene *GAPDH.*

### Digital light microscopy

The analyses can be performed with an epifluorescence microscope, e.g. KEYENCE BZ-8000, using a S-Plan-Fluor 20x lens. In the experiments described above, the filter sets ex560±40 - 630±60 nm/em (for red fluorescence) and ex480±30 - 535±50 nm/em (for green fluorescence) have been used. The samples are inspected by fluorescence microscopy. The overlays from both image series are computed.

### Scanning Electron Microscope

For scanning electron microscopy (SEM), a SU 8000 (Hitachi High-Technologies Europe) can be employed at low voltage (1 kV; suitable for analysis of inorganic morphological structures). For SEM observations, the beam deceleration mode is used to improve the scanning signals.

### Expression of recombinant proteins

The recombinant proteins (silicatein, silicase, and silintaphin-1) to be administered in combination with polyP or polyP (Ca²⁺ complex) can be prepared using state-of-the-art procedures.

The methods for preparation of the tagged proteins (tagged silicatein, tagged silicase, and tagged silintaphin-1) as well as the silicatein-silintaphin-1 fusion proteins have also been described (Silicatein: e.g., EP1320624, US7169589B2, EP10167744.1**,** EP1740707, US11579019, DE10352433.9, CA2565118, JP2007509991; Silintaphin-1: e.g., EP09005849.6, EP10167744.1).

For the purposes of the present invention, all references as cited are hereby incorporated by reference in their entireties. The legends to the Figures are as follows.
**Figure 1****.** Schematic presentation of the dual effect of polyP (Ca²⁺ complex) on osteoblasts and osteoclasts: (a) Promotion of osteoblast formation through upregulation of *BMP2* expression followed by increased HA deposition and (b) inhibition of maturation of osteoclast precursor cells to functional osteoclasts. In addition, the effect of (bio)silica [SiO₂] or silicic acid [(SiOH)_{4]} is shown. Si0₂ / (SiOH)₄ enhances the expression of *BMP2* and *OPG* in osteoblasts, resulting in an increased OPG : RANKL ratio. Thereby OPG sequesters RANKL and prevents its binding to RANK, leading to an impaired pre-osteoclast maturation and osteoclast activation and thus an inhibition of bone resorption.
**Figure 2****.** Influence of polyP (Ca²⁺ complex) on cell viability. SaOS-2 cells [white bars] or RAW 264.7 cells [black bars] were incubated with increasing concentrations of polyP (Ca²⁺ complex) for 5 days. Then the viability was measured with the XTT assay. The results are expressed as means (n=10 experiments each) ± SEM; **P* < 0.01.
**Figure 3****.** Induction of mineralization of SaOS-2 cells by polyP (Ca²⁺ complex) *in vitro.* SaOS-2 cells were cultured in McCoy's medium/FCS for 2 days and, after attachment, transferred to McCoy's medium /10% FCS and subsequently continued to grow for 7 days (A) in the absence [- activation cocktail] or (B to D) presence [+ activation cocktail] of AC (5 mM 13-glycerophosphate / 50 mM ascorbic acid / 10 nM dexamethasone) required for extensive HA formation. Where indicated the assays were supplemented with either 10 µM polyP [PP] or 10 µM polyP (Ca²⁺ complex) [PP/Ca]. Then the specimens were stained with AR-S and the samples were analyzed by digital light microscopy (rows a and c) or by red and green fluorescence light and documented as overlays (rows b and d). The red staining reflects the extent of HA mineralization. (E) In parallel plastic cover slips were likewise stained with AR-S and documented as a whole.
**Figure 4****.** Formation of HA deposits/nodules on SaOS-2 cells in medium supplemented with polyP or polyP (Ca²⁺ complex) during an incubation period of 5 days; SEM observations. (A) Cells (c) were grown in the absence of AC; no nodules were seen. (B) SaOS-2 cultivated in medium/AC and the presence of 10 µM polyP; frequently nodules (no) were found. (C and D) Mineralization of SaOS-2 cells (c) grown in medium/AC and 10 µM polyP (Ca²⁺ complex). Abundantly, HA nodules (no) were visualized which were mostly surrounded by cell protrusions.
**Figure 5****.** Formation of HA deposits/nodules on SaOS-2 cells in medium supplemented with polyP or polyP (Ca²⁺ complex) during an incubation period of 5 days; SEM observations. (A) Cells (c) were grown in the absence of AC; no nodules were seen. (B) SaOS-2 cultivated in medium/AC and the presence of 10 µM polyP; frequently nodules (no) were found. (C and D) Mineralization of SaOS-2 cells (c) grown in medium/AC and 10 µM polyP (Ca²⁺ complex). Abundantly, HA nodules (no) were visualized which were mostly surrounded by cell protrusions.
**Figure 6****.** Increased expression of *BMP2* in SaOS-2 cells in assays supplemented with polyP (Ca²⁺ complex). The technique of qRT-PCR was applied to determine the expression levels of *BMP2* and *GAPDH* (used as reference for normalization). Black bars represent *BMP2* expression of cells grown in the absence of polyP (Ca²⁺ complex) for 7 days; dark gray bars summarize the expression levels *of BMP2* in cells treated with 10 µM polyP (Ca²⁺ complex); and the light gray bars show the levels *of BMP2* in SaOS-2 cells treated with 100 µM polyP (Ca²⁺ complex). Standard errors of the means are shown (n = 5 experiments per time point); **P* < 0.01.
**Figure 7****.** Inhibitory effect of polyP (Ca²⁺ complex) on the differentiation of RAW 264.7 cells. The cells were incubated for 6 days with increasing concentrations of polyP (Ca²⁺ complex) together with 50 ng/ml of soluble RANKL. Then the cells were fixed and stained for TRAP. The percentage of TRAP⁺ cells is given. The mean values (± SD) from 6 independent assays are indicated (**P* < 0.01).

## Claims

1. Drug or food supplement or injectable material, comprising or consisting of inorganic polyphosphate (polyP) or complexes of polyP and calcium [polyP (Ca²⁺ complex)] or combinations thereof.

2. Material according to claim 1, wherein the chain length of the polyP molecules or of the polyP molecules of the polyP (Ca²⁺ complex) are in the range of 100 to 1000 phosphate units, preferably in the range 2 to 1000 phosphate units and more preferably 10 to 100 phosphate units.

3. Material according to claim 1 or 2, consisting of a combination of polyP or polyP (Ca²⁺ complex) and monomeric silicic acid (orthosilicic acid) or polymeric silicic acid (silica).

4. Material according to 3, wherein said monomeric or polymeric silicic acid is present as a calcium salt.

5. Material according to claim 3 or 4, wherein said monomeric or polymeric silicic acid is present in a stabilized form such as hydronium stabilized, choline stabilized, or protein-stabilized.

6. Material according to any of claims 3 to 5, wherein the polymeric silicic acid has been formed by an enzyme or protein involved in biosilica (amorphous, hydrated silicon oxide) metabolism, such as silicatein, silicase, silintaphin-1, silicatein-silintaphin-1 fusion proteins or combinations thereof.

7. Material according to any of claims 3 to 6, wherein silicatein, silicase or silintaphin-1 or silicatein-silintaphin-1 fusion proteins or combinations thereof, as well as a suitable substrate are present.

8. Material according to claim 6 or 7, wherein said enzyme or protein is provided with an N-terminal or C-terminal bound apatite (calcium phosphate) or silica-binding oligocationic or oligoanionic tag, or a multiple thiol groups carrying tag.

9. Material according to any of claims 3 to 8, wherein a silicatein polypeptide, a silicase polypeptide or a silicatein fusion protein is used, which has been produced using a prokaryotic or eukaryotic expression system, or which has been produced synthetically.

10. Material according to any of claims 1 to 9, wherein polyP or polyP (Ca²⁺ complex) or their salts, complexes or combinations with monomeric or polymeric silicic acid or one or more of the components (enzymes, proteins, and substrates) involved in their formation has/have been encapsulated in an organic polymer, wherein said organic polymer preferably consists of shellac, alginate, or poly(lactic acid), or poly(D,L-lactide)/polyvinyl pyrrolidone-based microspheres.

11. Material according to any of claims 1 to 10, **characterized in that** said polyP or polyP (Ca²⁺ complex) or their combinations are bound to silica or hydroxyapatite nanoparticles.

12. Material according to any of claims 1 to 11, formulated as an orally or parenterally-administrable drug for the therapy or prophylaxis of osteoporosis or other bone disorders, or formulated for percutaneous injection into fractured bone tissue or the prophylactic stabilization of vertebral bodies of osteoporotic patients or patients with other bone disorders.

13. Material according to any of claims 1 to 12, wherein said material is present as a supplement in yoghurt or other milk products.

14. Material according to any of claims 1 to 11 for use as a food supplement or for the prophylaxis or therapy of diseases, such as osteoporosis or other bone disorders.

15. Material according to any of claims 1 to 14, which is for combined administration with bisphosphonates for the prophylaxis or treatment of osteoporosis or other bone diseases.
